# EUROPÄISCHE PATENTSCHRIFT

(11) **EP 0 645 449 B1**
(45) Veröffentlichungstag und Bekanntmachung des Hinweises auf die Patenterteilung: **06.03.2002**
(21) Anmeldenummer: 94114675.5
(22) Anmeldetag: 17.09.1994
(51) Int. Cl.: C12N 15/10, C12Q 1/68

(54) **Verfahren zur spezifischen Klonierung von Nukleinsäuren**
Method for specific cloning of nucleic acids
Méthode pour le clonage spécifique d'acides nucléiques

(30) Priorität: 24.09.1993 DE 4332463
(43) Veröffentlichungstag der Anmeldung: 29.03.1995
(73) Patentinhaber: november Aktiengesellschaft Gesellschaft für Molekulare Medizin, 91056 Erlangen (DE)
(72) Erfinder: Bertling, Wolf, Dr., D-91056 Erlangen (DE)
(74) Vertreter: Gassner, Wolfgang, Dr.

(56) Entgegenhaltungen:
- WO-A-91/03573
- WO-A-91/18114
- FR-A- 2 678 639
- US-A- 4 666 839
- PCR METHODS APPL. 3(2), 95-9, Oktober 1993 Bertling, Wolf M. et al 'Determination of 5' ends of specific mRNAs by DNA ligase-dependent amplification'

## Beschreibung

Die Erfindung betrifft ein Verfahren zur Klonierung von Nukleinsäuren mittels Ligation einer einzelsträngigen DNA unter Verwendung zweier teilweise komplementärer, doppelsträngiger Oligonukleotide, Synthese des zweiten Stranges sowie gegebenenfalls gezielte Amplifikation der Ziel-DNA mittels zweier spezifischer Oligonukleotide und Reagenzien zu deren Durchführung.

Nukleinsäuren sind für alle bisher bekannten Organismen als Informationsträger die Grundlage spezifischer Lebensformen. Sie kodieren für Proteine oder haben auch katalytische oder strukturelle Wirksamkeiten. Eine Reihe von Techniken zum Klonieren und Vermehren von Nukleinsäuren sind für Desoxyribonukleinsäure (DNA) etabliert. Das Klonieren von Ribonukleinsäuren (RNA) hingegen ist besonders für die Klonierung von selten vorkommenden RNAs und für die Klonierung von RNAs in voller Länge noch relativ schwierig.

Bestehende Methoden für die RNA Klonierung basieren auf der Isolierung von Gesamt- oder mRNA aus Zellen, der Übersetzungen der RNA in ein cDNA: RNA-Hybrid mittels reverser Transkriptasen (AMV, MoMuLV), Abbau des RNA-Anteils mit RNase H, gefolgt von der Synthese des komplementären cDNA Stranges mit Klenow DNA Polymerase (als Standardmethoden beschrieben in Ausubel et al) (Editor) 1987 Current Protocols in Molecular Biology). Die Synthese des zweiten Strangs der DNA benötigt einen Primer. Der kann aus kurzen RNA Fragmenten bestehen, die von RNase H Verdau resultieren (Gubler 1987, Methods of Enzymology 152, 330 - 335), er kann, wie in der klassischen Methode, durch eine Rückfaltung der einzelsträngigen cDNA gegeben sein (Gubler, 1987, Methods of Enzymology 152, 325 - 329) oder auch durch Zugabe von hoch degenerierten kurzen Oligonukleotiden entstehen (Feinberg, Vogelstein, 1983 Analytical Biochemistry 132, 6 - 13). In allen Fällen geht in der Regel das 3' Ende der einzelsträngigen cDNA bei der Klonierung verloren, da mangels Primer kein komplementärer Strang synthetisiert wird. Die so erzeugte doppelsträngige DNA kann dann über die Klonierung von geeigneten Adaptoren spezifisch in Vektoren (Plasmide, Cosmide, Phagen) kloniert und anschließend effizient in Bakterien transformiert oder in Phagen verpackt werden.

Durch die Entwicklung der Polymerase-Kettenreaktion (PCR) (Saiki et al, Science 1988, 239, 487 - 491) konnte eine weitere Verbesserung der Klonierung von RNAs über die aufgezeigte Methode erzielt werden. Die Kombination dieser beiden Methoden erlaubt die Klonierung von extrem geringen Ausgangsmengen an RNA (geringe Menge an Ausgangszellen) oder die Klonierung von extrem selten vorkommender RNA.

Die Verwendung von PCR in der cDNA-Klonierung setzt voraus, daß die cDNA-sowohl an Ihrem 3' Ende als auch an ihrem 5' Ende DNA Regionen mit bekannter Nukleotidsequenz aufweist Eine Sequenz mit bekannter Nukleotidfolge kann durch ein Oligonukleotid mit poly-dT Schwanz, der komplementär zum 3' Ende der mRNA ist, relativ einfach an das 5' Ende der cDNA eingefügt werden.

Weitaus schwieriger ist die Verankerung einer spezifischen Sequenz am 3' Ende der cDNA. Die bisher beschriebenen Methoden sind:

Synthese eines Homopolymers (z.B. dG oder dA) mit terminaler Transferase. Diese Methode ist als "Rapid Amplification of cDNA Ends" (RACE) oder "Anchored PCR" bekannt geworden (Frohman et al, 1988, PNAS 85, 8998 - 9002). Diese Methode beruht darauf, daß an das 3' Ende der cDNA ein Homopolymer ansynthetisiert wird und anschließend eine PCR durchgeführt wind, wobei ein Primer komplementär zum neu synthetisierten 3' Homopolymer-Ende der Einzelstrang cDNA ist, der zweite Primer ist homolog einem Bereich des 5' Endes der cDNA.

Zwei weitere Methoden, die ebenfalls einen Amplifikationsschritt enthalten, sind in den letzten Jahren veröffentlicht worden. Beide Methoden verwenden T4 RNA Ligase. Diese Ligase oder eine andere einzelstrangspezifische Ligase wird entweder dazu verwendet, um an das 3' Ende der cDNA einen Primer zu "verankern". Zwei weitere Primer, einer homolog zu einem weiter 5' liegenden Bereich des reversen Transkripts, der einzelstängigen cDNA, und einer komplementär zu dem anligierten Primer, werden dann in einer nachfolgenden Amplifikation (PCR) eingesetzt (Troutt et al, 1992, PNAS 89, 9823 -9825) (Dymas Mallet für Rhône Poulenc Rorer SA, französisches Patent No. 9108294). Alternativ dazu können die durch reverse Transkription entstandenen einzelsträngigen DNAs jeweils über ihre 5' bzw. 3' Enden miteinander verbunden werden (head-to-tail), so daß in einer anschließenden Amplifikation zwei spezifische Primer, einer homolog und einer komplementär zu einer vorgegebenen Sequenz der zu amplifizierenden cDNA, eingesetzt werden können (Hofmann, Brian 1991, PCR Methods and Application 1, 43 -45).

Die WO-A-9103573 beschreibt ein Verfahren zur Vermehrung von Nukleinsäuresequenzen, welches auf der Verwendung mindestens zweier Adaptoren pro zu vermehrender Nukleinsäuresequenz beruht. Die Adaptoren sind zu unterschiedlichen Bereichen der zu vermehrenden Nukleinsäuresequenz komplementär. Die Nukleinsäuresequenz des einzelsträngigen hybridisierenden Bereichs der Adaptoren ist so gewählt, daß mindestens ein Adaptor mit einem einzelsträngigen 5'-Ende und ein weiterer Adaptor mit einem einzelsträngigen 3'-Ende beteiligt ist.

Alle beschriebenen Techniken zeigen Nachteile, die ihre Anwendung limitieren. Besonders hervorzuheben ist, daß die Stufen, die Primer verwenden, nicht immer korrekt kontrollierbar sind und die Spezifität der Primer, speziell im Fall der Homopolymere limitiert ist. Das führt nicht nur zu beträchtlichen Hintergrundsignalen, sondern bedingt zumindest bei den nicht auf Amplifikation beruhenden Techniken, aber auch bis zu einem gewissen Grad bei den PCR- abhängigen Schritten zu Produkten, die die Sequenzinformation des 5'-Endes der RNA (meist mRNA) nicht enthalten. Zumindest die nicht auf Amplifikation beruhenden Verfahren lassen auch die Erstellung von Genbanken nur sehr bedingt zu, da nicht aus allen Zellen und Gewebsproben genügend RNA isolierbar ist, um diese Prozeduren durchzuführen und die erhaltenen doppelsträngigen DNA Fragmente erfolgreich zu klonieren.

Die Methode, die unter Synthese eines Homopolymers verläuft, ist sehr stark limitiert dadurch, daß die terminale Transferase nicht sehr effizient dG oder dA aber auch keine langen Homopolymerketten mittels dC oder dT synthetisiert und die Reaktion schlecht zu kontrollieren ist.

Ein weiterer Nachteil wird bei der darauffolgenden Amplifikation ersichtlich. Die verwendeten Primer (z.B. homopolymere dC oder dA Primer) neigen zur Bildung von unspezifischen Amplifikationsprodukten, was den Hintergrund an unerwünschten klonierten Fragmenten erheblich erhöht.

Die anderen amplifikationsabhängigen Methoden verwenden Enzyme, die in der Lage sind, einzelsträngige Moleküle zu ligieren, vorzugsweise T4 RNA Ligase. Diese Ligase neigt dazu, kurze Oligonukleotide und Fragmente (von RNA und DNA) zu ligieren und die Ausbeute an konkatameren, einzelsträngigen cDNAs ist relativ gering. Entsprechend gering ist dann auch die Ausbeute an Amplifikationsprodukten.

Oligonukleotidprimer wiederum werden nicht nur an cDNA ligiert, sondern ebenso an RNA und kurze Fragmente von cDNA und RNA. Solche Ligationsprodukte erhöhen den Hintergrund an falsch amplifizierten Produkten und erfordern sehr aufwendige Reinigungsschritte, die wiederum mit Materialverlust einhergehen.

Die genannten Probleme werden durch das erfindungsgemäße Verfahren gelöst. Es handelt sich im einzelnen dabei um ein Verfahren zur Klonierung von Nukleinsäuren mittels Ligation einer einzelsträngigen DNA unter Verwendung zweier teilweise komplementärer, und damit doppelsträngiger Oligonukleotide, der Möglichkeit daran die Synthese des zweiten Stranges einzuschließen sowie der möglichen gezielten Amplifikation der Ziel-DNA mittels zweier spezifischer Oligonukleotide anzuhängen. Eine bevorzugte Anwendung der Erfindung ist das Herstellen von Klonen, besonders von Klonen, die das komplette 5' Ende von mRNAs repräsentieren. Die in diesem Verfahren verwendeten Produkte sichern eine hohe Spezifität der Produkte. Ob und bis zu welchem Grad Amplifikation des Produkts des Ligationsschritts gewünscht wird, kann frei bestimmt werden.

Eine Anwendung der Erfindung besteht daher in einem Klonierungsschema, das durch folgende Schritte charakterisiert ist:
- In einem ersten Schritt wird eine einzelsträngige DNA oder auch doppelsträngige DNA mit einzelsträngigem Anteil für die weiteren Schritte bereit gestellt, hergestellt, gewonnen oder anderweitig erworben (Ziel-DNA). Bei der Erstellung aus RNA kann es sich als besonders günstig erweisen, ein Enzym zu verwenden, das bei höheren Temperaturen arbeiten kann.
- In einem zweiten Schritt werden die für den jeweiligen Versuch benötigten Primer bereit gestellt, hergestellt, gewonnen oder anderweitig erworben. Dabei ist darauf zu achten, daß der an das 3' Ende der DNA zu ligierende Primer an seinem 5' Ende phosphoryliert vorliegt.
- In einem weiteren Schritt werden ein Gemisch zweier, teilweise komplementärer Primer (Oligonukleotide oder evtl. Polynukleotide), von denen mindestens und bevorzugt einer an einem Ende über den komplementären Bereich hinausreicht (Überhang), vorbereitet. Dieser Überhang stellt einen degenerierten Bereich dar, das heißt, an bevorzugter Weise jeder möglichen Position befindet sich eins der klassischen Nukleotide oder auch ein modifiziertes Nukleotid mit Desoxy-Inosin. Daraus ergibt sich im Gemisch die bevorzugterweise gleiche Repräsentation jeder verwendeten Base an jeder Position des Überhangs. Dieses so beschriebene Gemisch vom Primern wird im folgenden Primerpaar genannt.
- In einem weiteren Schritt wird die Ligation des Primerpaars an die Ziel-DNA vorbereitet und durchgeführt. Die beiden letzten Schritte können auch gemeinsam durchgeführt werden. Es läßt sich empfehlen, vor dem Ansetzen der Ligation überschüssige Primer, z.B. durch Gelfiltration, zu entfernen.
- In einem weiteren Schritt kann die Amplifikation des Ligationsprodukts durchgeführt werden, evtl. nach vorausgegangener Reinigung, Verdünnung oder Konzentrierung des Ligationsansatzes. Die Amplifikationstemperaturen sollen dabei analog der bekannten Maßnahmen (US. 4.683.195 und 4.683.202) gewählt werden.
   Aufgrund seiner Spezifität für doppelsträngige Bereiche von DNA ist ein bevorzugtes Enzym für die Ligationsreaktion die T4 DNA Ligase. Es empfiehlt sich, die Ligation bei 15°C bis 25°C über einen Zeitraum von 1 - 16 Stunden durchzuführen. Üblicherweise werden dabei kürzere Zeiten mit höheren Temperaturen und längere Inkubationszeiten mit niedrigeren Temperaturen kombiniert. Vorzugsweise wird die Ligation bei 16°C über Nacht durchgeführt. Es stehen neuerdings auch thermostabile Ligasen zur Verfügung. Bei der Auswahl der zu verwendenden Oligonukleotide empfiehlt es sich, sowohl auf eine ausreichende Länge, ein adäquates G/C-Verhältnis und auch die Verfügbarkeit von Restriktionsendonuklease Erkennungsstellen zu achten, die bei einer späteren Klonierung verwendet werden können.

In abschließenden Schritten findet die Vorbereitung des Amplifikates für eine anschließende Weiterbehandlung, z.B. zur Sequenzierung oder Klonierung statt. Es kann sich auch als angebracht erweisen, das Amplifikationsprodukt einer weiteren sogenannten "Nested" PCR zu unterwerfen, um z.B. den Hintergrund zu vermindern oder die Spezifität zu erhöhen. Bei der ersten Amplifikation wird meist, z.B. aus Kostengründen, derselbe Primer als sogenannter Downstream Primer verwendet, wie für die reverse Transkription. Bei der zweiten, "Nested" Amplifikation wird nun ein Downstream Primer gewählt, der nach oben verschoben, d.h. näher am 3' Ende der einzelsträngigen DNA gelegen ist. Prinzipiell ist bei Verwendung geeigneter Enzyme auch die sukzessive Durchführung aller enzymatischen Reaktionen in einem Gefäß möglich.

Bevorzugt wird die Erfindung auf einzelsträngige cDNA angewandt, die durch reverse Transkription von mRNA entstanden ist. Die Herstellung von RNA, sowie die reverse Transkription dieser RNA wird nach bekannten und beschriebenen Protokollen stattfinden (z.B. Ausubel et al 1987 (Editor) Current Protocols of Molecular Biology). Es ist für sämtliche Primer; außer dem, der an das 3' Ende der DNA ligiert werden soll, nicht nötig, sondern eventuell sogar nachteilig, am 5' Ende phosphoryliert zu sein.

Durch einen zusätzlichen Schritt läßt sich auch genomische DNA als Vorlage für die Ligation an das Primerpaar verwenden. In diesem Schritt wird genomische DNA mittels eines Primers, der komplementär oder homolog zu einem bekannten Teil der DNA ist, als Vorlage für eine Extension mittels einer DNA-abhängigen DNA-Polymerase bzw. einer DNA-abhängigen RNA-Polymerase (falls der Primer entsprechende Promotorsequenzen, z.B. für den RNA-Polymerase des Phagen SP6, enthält) in einzelsträngige DNA bzw. RNA überführen. Durch Aufreinigen der einzelsträngigen Produkte, z.B. über eine für einzelsträngige Moleküle spezifische (z.B. Hydroxylapatit) oder RNA spezifische Säule oder auch über eine Affinitätsaufreinigung, falls z.B. der verwendete Primer eine für dieses Verfahren geeignete Modifikation (z.B Biotin, Digoxigenin) enthält, läßt sich auch daraus ein geeignetes Substrat für die anschließende Ligation erhalten.

Es ist für manche Anwendungen wünschenswert, modifizierte Nukleotide, z.B. m⁵dC (an Position 5 methyliertes Desoxycytonsin) zu verwenden, die zum Erhalt eines Produkts führen, das resistent gegen Restriktionsendonukleasen ist.

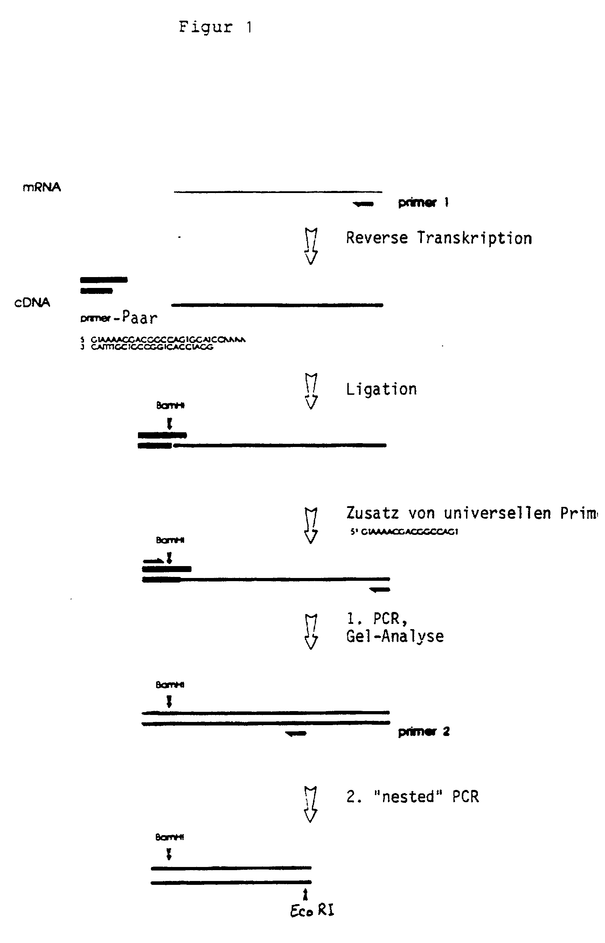

**Figur 1**: Gibt einen Überblick über das Prinzip der Beispiele 1 - 4. Nach reverser Transkription wurde das Primerpaar an die einzelsträngige cDNA ligiert, anschließend mit dem Primer, der für die reverse Transkription verwendet worden war, und einem, der komplementär zu einem Teil des an die cDNA ligierten Primers war, einer PCR unterworfen. Nach Aufreinigung des Amplifikationsproduktes wurde eine zweite PCR durchgeführt mit dem gleichen upstream, aber einem anderen downstream Primer, der aber homolog zu einer Sequenz, die näher am 3'Ende der ursprünglichen cDNA gelegen war. Das daraus resultierende Amplifikationsprodukt wurde danach noch für die Klonierung vorbereitet.

### Beispiele:

Abbildung 1 gibt einen Überblick über das Prinzip des Beispiels 1 und 2 sowie Beispiel 3 und 4. Nach reverser Transkription wurde das Primerpaar an die einzelsträngige cDNA ligiert, anschließend mit dem Primer, der für die reverse Transkription verwendet worden war, und einem, der komplementär zu einem Teil des an die cDNA ligierten Primers war, einer PCR unterworfen. Nach Aufreinigung des Amplifikationsprodukts wurde eine zweite PCR durchgeführt mit dem gleichen Upstreamprimer, aber einem anderen, ebenfalls spezifischen. aber homolog zu einer Stelle, die näher am 3' Ende der ursprünglichen cDNA gelegen war, durchgeführt. Das daraus resultierende Amplifikationsprodukt wurde danach noch für die Klonierung vorbereitet.

### Beispiel 1:

### Herstellung einer spezifischen cDNA durch reverse Transkription.

RNA wurde präpariert wie beschrieben (Ausubel et al 1987). Oligonukleotide wurden an einem Gene-Assembler plus (Pharmacia) synthetisiert und nach Detritylierung gereinigt durch Zentrifugation durch eine G 50 Säule.

Ein spezifischer Primer (30 pmol), der komplementär zur mRNA war, wurde in einem 30 µl reversen Transkriptionsansatz eingesetzt. Dieser Ansatz verlief in folgenden Puffer:
10 mM Tris HCl (pH 8,3), KCl 50 mM, MgCl₂ 5 mM, dNTP (dATP, dCTP, dGTP, dTTP) je 1 mM. Der Ansatz enthielt ferner 3 µg Gesamt-RNA, 40 units RNAsin und 50 units MoMuLV reverser Transkriptase und wurde auf 30 µl mit sterilem bidestillierten Wasser aufgefüllt. Die reverse Transkription wurde für 15 Min bei 42°C durchgeführt. Danach wurden die Nukleinsäuren mit Ethanol gefällt, in 350 µl RNase Verdaupuffer aufgenommen (Tris HCl (pH 7,5) 10 mM, NaCl 300 mM, EDTA 5 mM). Die verbliebene RNA wird durch Zugabe von RNase A (1,5 units) und RNase T1 (700 units) bei 37°C 30 Min lang verdaut. Das im Ansatz enthaltene cDNA Produkt wurde durch eine Zentrifugationsäule (Sephadex G50) gereinigt.
Nach Verkleinern des Volumens des cDNA enthaltenden Eluats durch Lyophilisation und Ethanolpräzipitation wurde das Produkt in 6 µl sterilem bidestilliertem Wasser aufgefüllt.

### Beispiel 2:

### Ligation eines Primerpaars an eine einzelsträngige Ziel-DNA.

Die 2 für die Ligation nötigen Primer (das Primerpaar) sind in ihrer Sequenz in Abbildung 1 wiedergegeben. Der Primer, der mit seinem 5' Ende an das 3' Ende der cDNA von Beispiel 1 ligiert werden sollte, lag phosphoryliert vor.

Die Ligation wurde in 10 µl mit 10 pmol jedes Primers bzw. Primergemisches bei 16 °C über Nacht ausgeführt in einer Lösung, die Tris HCl (pH 7,5) 66 mM, MgCl₂ (5 mM) DTT (1 mM) und ein Drittel der in Beipiel 1 dargestellten cDNA enthielt. Nach der Inkubation wurde der Ansatz 5 Min lang bei 70°C gehalten, dann auf Raum-temperatur abgekühlt und durch Zugabe von ATP (10 nmol) und T4 DNA Ligase (Boehringer Mannheim GmbH) (1 unit) gestartet.

### Beispiel 3:

### Amplifikation eines Aliquots der Ligationsreaktion.

Die Amplifikation fand in 100 µl eines Puffergemisches (10 mM Tris HCl (pH 8,3), 50 mM KCl, 2 mM MgCl₂, 0,1 mg/ml Gelatine) unter Verwendung von je 40 nmol der Nukleotide dATP, dCTP, dGTP, dTTP und je 50 pmol der beiden Primer (des für die reverse Transkription verwendeten und eines weiteren, der komplementär zum anligierten Primer war) statt. In dem Ansatz befanden sich ferner 40 % des Ligationsansatzes (Beispiel 2) und 2 units Taq Polymerase. Die Amplifikation erfolgte in 25 Runden durch Durchlaufen des Zyklus: 94°C 45 Sek. 42°C 30 Sek, 72°C 90 Sek.

### Beispiel 4:

### Zweite, "Nested" Amplifikation des Amplifikationsprodukts aus Beispiel 3.

20 % des PCR-Ansatzes aus Beispiel 3 wurde auf einem 1,5 %igem Agarosegel elektrophoretisch getrennt (Ausubel et al), die Bande der erwarteten Größe ausgeschnitten, und einer Aufreinigung mit Glassmilk (GeneClean, Bio 101, CA, USA) unterzogen. Die so eluierte DNA lag danach in 30 µl sterilem bidestilliertem Wasser vor. 5 µl davon wurden für eine erneute Amplifikation ausgewählt, die unter den gleichen Bedingungen, wie in Beispiel 3 durchgeführt wurde, mit dem Unterschied, daß diesmal nicht der für die reverse Transkription verwendete Primer, sondern statt dessen ein ebenfalls für die getestete RNA spezifischer Primer verwendet wurde, der dem 3' Ende der ursprünglichen cDNA näher lag. Selbstverständlich erfolgte kein neuer Zusatz eines Aliquots des Ligationsansatzes. Aliquots dieses Amplifikationsansatzes wurden, wie oben beschrieben, gelelektrophoretisch analysiert (sowohl vor, als auch nach Verdau mit passenden Restriktionsendonukleasen). Nach Aufreinigung (siehe oben) der Verdauprodukte lagen diese in einer für weitere Klonierungsschritte geeigneten Form vor.

## Patentansprüche

1. Verfahren zur Klonierung von Nukleinsäuren mit unbekanntem 3'-Ende, wobei
in einem ersten Schritt die Ligation einer doppelsträngigen Kombination von zweien, zueinander teilweise komplementären Primern an das 3'-Ende eines einzelsträngigen DNA-Strangs durchgeführt wird, wobei ein Primer an einem Ende mit einem einen degenerierten Bereich darstellenden Überhang über den komplementären Bereich hinausreicht, wobei die Ligation durch die Gegenwart eines Enzyms ausgelöst wird mit Spezifität für doppelsträngige DNA,
in einem zweiten Schritt die Synthese des zweiten Strangs mittels der DNA-Polymerase durchgeführt wird, wobei ein Primer verwendet wird, der zumindest zu einem Teil des anligierten Oligonukleotids komplementär ist und
gegebenenfalls in einem dritten Schritt die Amplifikation der so erhaltenen cDNA durchgeführt wird.

2. Verfahren gemäß Anspruch 1, charakterisiert dadurch, daß die einzelsträngige DNA durch reverse Transkription einer Matrizen-RNA entstanden ist.

3. Verfahren gemäß Anspruch 1, charakterisiert dadurch, daß die einzelsträngige DNA durch Bearbeitung einer Matrizen-DNA entstanden ist.

4. Verfahren gemäß Anspruch 2 und 3 unter Verwendung von modifizierten Oligos bei der Zweitstrangsynthese und in der PCR, die zum Erhalten eines Produkts führen, das resistent gegen Restriktionsendonukleasenverdau ist.

5. Verfahren gemäß Anspruch 4 unter Verwendung von m⁵C als modifizierte Base in einem Oligonukleotid.

6. Verfahren gemäß Anspruch 3, **dadurch gekennzeichnet, daß** die Abtrennung der durch Primerextension dargestellten cDNA erfolgt.

7. Verfahren gemäß Anspruch 6, **dadurch gekennzeichnet, daß** die Abtrennung über Affinitätschromatographie erfolgt.

8. Verfahren gemäß Anspruch 7, **dadurch gekennzeichnet, daß** die Abtrennung durch modifizierte Primer bzw. Oligonukleotide ermöglicht wird.

9. Verfahren gemäß Anspruch 8, **dadurch gekennzeichnet, daß** ein biotinylierter oder digoxigenierter, markierter Primer dafür eingesetzt wird.

10. Verfahren gemäß Anspruch 1, charakterisiert dadurch, daß man Oligonukleotide, Primer verwendet, die zur Erzeugung von Restriktionsenzymserkennungsstellen im Produkt führen.

11. Verfahren gemäß Anspruch 1, **dadurch gekennzeichnet, daß** der Überhang des Primerpaars mehr als drei und weniger als 6 Nukleotide umfaßt.

12. Verfahren gemäß Anspruch 1, charakterisiert dadurch, daß die Ligation durch die Gegenwart eines Enzyms ausgelöst wird, mit Spezifität für doppelsträngige DNA.

13. Verfahren gemäß Anspruch 12, charakterisiert dadurch, daß es sich bei diesem Enzym um T4 DNA Ligase handelt.

14. Verfahren gemäß Anspruch 12, charakterisiert dadurch, daß es sich dabei um eine thermostabile Ligase handelt.

15. Verfahren gemäß Anspruch 2, charakterisiert dadurch, daß ein thermostabiles Enzym für die reverse Transkription verwendet wird.

16. Verfahren gemäß Anspruch 15, charakterisiert dadurch, daß es sich dabei um eine DNA Polymerase mit reverser Transkriptase Aktivität handelt; z.B. Tth DNA Polymerase.

17. Verfahren gemäß Anspruch 1, charakterisiert dadurch, daß die Gewinnung der doppelsträngigen DNA durch eine DNA abhängige DNA Polymerase geschieht.

18. Verfahren gemäß Anspruch 17, charakterisiert dadurch, daß es sich dabei um das Klenow Fragment der DNA Polymerase von E. coli handelt.

19. Verfahren gemäß Anspruch 17, **gekennzeichnet dadurch, daß** es sich dabei um eine thermostabile DNA Polymerase handelt.

20. Verfahren gemäß Anspruch 19, **gekennzeichnet dadurch, daß** es sich dabei um Taq Pol handelt.

21. Verfahren gemäß Anspruch 17, **gekennzeichnet dadurch, daß** dafür modifizierte Primer verwendet werden, die eine Aufreinigung der entstehenden einzelsträngigen DNA zulassen.

22. Verfahren gemäß Anspruch 21, **gekennzeichnet dadurch, daß** es sich bei der Modifikation um Biotin und bei der Affinitätsmatrix um Avidin oder Streptavidin oder bei der Modifikation um Digoxigenin und bei der Affinitätsmatrix um Anti DIG AK handelt.

23. Verfahren gemäß Anspruch 12, **gekennzeichnet dadurch, daß** die Ligationstemperatur zwischen 15° und 25°C liegt und die Ligation für eine, Dauer von 1 bis 16 Stunden durchgeführt wird.

24. Verfahren gemäß Anspruch 1, charakterisiert dadurch, daß im dritten Schritt ein weiterer, neuer Primer, komplementär zu einem Teil des an die Einzeistrang-DNA ligierten Sequenzbereichs, verwendet wird.

25. Verfahren gemäß Anspruch 24, charakterisiert dadurch, daß anschließend mehrere Amplifikationszyklen durchgeführt werden.

## Claims

1. Process for cloning nucleic acids with unknown 3'-end, wherein:
in a first step a ligation of a double-stranded combination of two primers that are partially complementary to one another to the 3' end of a single-stranded DNA strand is carried out, wherein one primer at one end extends beyond the complementary region with an overhang representing a degenerated region, wherein the ligation is triggered in presence of an enzyme being specific for double-stranded DNA
in a second step the second strand is synthesized by means of a DNA polymerase in which a primer is used which is complementary to at least part of the ligated oligonucleotide and
if desired, the cDNA obtained in this manner is amplified in a third step.

2. Process as claimed in claim 1, wherein the single-stranded DNA is formed by reverse transcription of a template RNA.

3. Process as claimed in claim 1, wherein the single-stranded DNA is formed by processing a template DNA.

4. Process as claimed in claim 2 and 3 using modified oligos in the second strand synthesis and in the PCR which lead to a product being formed that is resistant to digestion by restriction endonucleases.

5. Process as claimed in claim 4 using m⁵C as a modified base in an oligonucleotide.

6. Process as claimed in claim 3, wherein the cDNA prepared by primer extension is separated.

7. Process as claimed in claim 6, wherein the separation is carried out by means of affinity chromatography.

8. Process as claimed in claim 7, wherein the separation is made possible by modified primers or oligonucleotides.

9. Process as claimed in claim 8, wherein a biotinylated or digoxigenylated, labelled primer is used for this.

10. Process as claimed in claim 1, wherein oligonucleotides are used as the primers which lead to the generation of restriction enzyme recognition sites in the product.

11. Process as claimed in claim 1, wherein the overhang of the primer pair comprises more than three and less than 6 nucleotides.

12. Process as claimed in claim 1, wherein the ligation is triggered by the presence of an enzyme that is specific for double-stranded DNA.

13. Process as claimed in claim 12, wherein this enzyme is T4 DNA ligase.

14. Process as claimed in claim 12, wherein it is a thermostable ligase.

15. Process as claimed in claim 2, wherein a thermostable enzyme is used for the reverse transcription.

16. Process as claimed in claim 15, wherein it is a DNA polymerase with reverse transcriptase activity e.g. Tth DNA polymerase.

17. Process as claimed in claim 1, wherein the double-stranded DNA is produced by a DNA-dependent DNA polymerase.

18. Process as claimed in claim 17, wherein it is the Klenow fragment of the DNA polymerase from E. coli.

19. Process as claimed in claim 17, wherein it is a thermostable DNA polymerase.

20. Process as claimed in claim 19, wherein it is Taq Pol.

21. Process as claimed in claim 17, wherein modified primers are used for this which allow a purification of the single-stranded DNA that is formed.

22. Process as claimed in claim 21, wherein the modification is biotin and the affinity matrix is avidin or streptavidin or the modification is digoxigenin and the affinity matrix is anti-DIG antibody.

23. Process as claimed in claim 12, wherein the ligation temperature is between 15° and 25°C and the ligation is carried out for a period of 1 to 16 hours.

24. Process as claimed in claim 1, wherein a further, new primer complementary to part of the sequence region ligated to the single-stranded DNA is used in the third step.

25. Process as claimed in claim 24, wherein several amplification cycles are subsequently carried out.

## Revendications

1. Procédé de clonage d'acides nucléiques à extrémité 3' inconnue, dans lequel
dans une première étape, on effectue la ligature d'une combinaison bicaténaire de deux amorces en partie complémentaires l'une de l'autre, à l'extrémité 3' d'un brin d'ADN monocaténaire, l'une des amorces s'étendant à une extrémité par une extension représentant une région dégénérée au-delà de la région complémentaire, la ligature étant déclenchée par la présence d'un enzyme ayant une spécificité pour un ADN bicaténaire,
dans une deuxième étape, on effectue la synthèse du second brin au moyen de l'ADN-polymérase, en utilisant une amorce qui est complémentaire au moins d'une partie de l'oligonucléotide ligaturé et
le cas échéant dans une troisième étape, on effectue l'amplification de l'ADNc ainsi obtenu.

2. Procédé suivant la revendication 1, **caractérisé en ce que** l'ADN monocaténaire est produit par transcription inverse d'un ARN matriciel.

3. Procédé suivant la revendication 1, **caractérisé en ce que** l'ADN monocaténaire est produit par traitement d'un ADN matriciel.

4. Procédé suivant les revendications 2 et 3, impliquant l'utilisation d'oligonucléotides modifiés dans la synthèse double brin et dans la réaction en chaîne de la polymérase, qui mènent à l'obtention d'un produit qui résiste à la digestion par des endonucléases de restriction.

5. Procédé suivant la revendication 4, impliquant l'utilisation de m⁵C comme base modifiée dans un oligonucléotide.

6. Procédé suivant la revendication 3, **caractérisé en ce que** la séparation de l'ADNc préparé par extension d'une amorce est effectuée.

7. Procédé suivant la revendication 6, **caractérisé en ce que** la séparation est effectuée par chromatographie d'affinité.

8. Procédé suivant la revendication 7, **caractérisé en ce que** la séparation est rendue possible par des amorces, autrement dit des oligonucléotides modifiés.

9. Procédé suivant la revendication 8, **caractérisé en ce qu'**on utilise pour sa mise en oeuvre une amorce marquée à la biotine ou à la digoxigénine.

10. Procédé suivant la revendication 1, **caractérisé en ce qu'**on utilise comme amorces des oligonucléotides qui génèrent des sites de reconnaissance d'enzymes de restriction dans le produit.

11. Procédé suivant la revendication 1, **caractérisé en ce que** l'extension de la paire d'amorces comprend plus de trois et moins de 6 nucléotides.

12. Procédé suivant la revendication 1, **caractérisé en ce que** la ligature est déclenchée par la présence d'un enzyme doué de spécificité pour un ADN bicaténaire.

13. Procédé suivant la revendication 12, **caractérisé en ce que** l'enzyme en question consiste en ADN-ligase de T4.

14. Procédé suivant la revendication 12, **caractérisé en ce que** l'enzyme est une ligase thermostable.

15. Procédé suivant la revendication 2, **caractérisé en ce qu'**on utilise un enzyme thermostable pour la transcription inverse.

16. Procédé suivant la revendication 15, **caractérisé en ce que** l'enzyme est une ADN-polymérase douée d'activité de transcriptase inverse, par exemple l'ADN-polymérase Tth.

17. Procédé suivant la revendication 1, **caractérisé en ce que** l'obtention de l'ADN bicaténaire s'effectue par une ADN-polymérase dépendant de l'ADN.

18. Procédé suivant la revendication 17, **caractérisé en ce qu'**il s'agit en l'occurrence du fragment de Klenow de l'ADN-polymérase de E. coli.

19. Procédé suivant la revendication 17, **caractérisé en ce qu'**il s'agit en l'occurrence d'une ADN-polymérase thermostable.

20. Procédé suivant la revendication 19, **caractérisé en ce qu'**il s'agit de la Taq-polymérase.

21. Procédé suivant la revendication 17, **caractérisé en ce qu'**on utilise des amorces modifiées à cet effet, qui permettent une purification de l'ADN monocaténaire produit.

22. Procédé suivant la revendication 21, **caractérisé en ce qu'**il s'agit de biotine pour la modification et d'avidine ou de streptavidine pour la matrice d'affinité, ou bien de digoxigénine pour la modification et d'anti-DIG AK pour la matrice d'affinité.

23. Procédé suivant la revendication 12, **caractérisé en ce que** la température de ligature se situe entre 15 et 25°C et la ligature est conduite pendant une durée de 1 à 16 heures.

24. Procédé suivant la revendication 1, **caractérisé en ce qu'**on utilise dans la troisième étape une autre nouvelle amorce complémentaire d'une partie de la région de séquence ligaturée à l'ADN monocaténaire.

25. Procédé suivant la revendication 24, **caractérisé en ce qu'**on conduit ensuite plusieurs cycles d'amplification.
